(19) Europäisches Patentamt
European Patent Office
Office européen des brevets

(11) Veröffentlichungsnummer: **0 191 286 B1**

(12) # EUROPÄISCHE PATENTSCHRIFT

(45) Veröffentlichungstag der Patentschrift: **07.08.91**

(51) Int. Cl.[5]: **A61K 7/06**, C07C 65/21

(21) Anmeldenummer: **86100133.7**

(22) Anmeldetag: **07.01.86**

(54) **Sebosuppressive kosmetische Mittel, enthaltend Alkoxy- oder Alkylbenzyloxy-benzoesäuren oder deren Salze.**

(30) Priorität: **14.01.85 DE 3500972**

(43) Veröffentlichungstag der Anmeldung:
**20.08.86 Patentblatt 86/34**

(45) Bekanntmachung des Hinweises auf die Patenterteilung:
**07.08.91 Patentblatt 91/32**

(84) Benannte Vertragsstaaten:
**AT BE CH DE FR GB IT LI NL SE**

(56) Entgegenhaltungen:
**EP-A- 0 054 174**
**EP-A- 0 114 051**
**EP-A- 0 135 433**
**WO-A-82/04189**
**US-A- 3 716 644**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Möller, Hinrich, Dr.**
**Schumannstrasse 11**
**W-4019 Monheim(DE)**
Erfinder: **Wallat, Siegfried, Dr.**
**Marie-Curie-Strasse 9**
**W-4019 Monheim(DE)**

EP 0 191 286 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft die Verwendung topischer, kosmetischer Mittel, die bestimmte Alkoxy- oder Alkylbenzyloxy-benzoesäuren oder deren Salze enthalten, zur Verbesserung des fettigen und unästhetischen Aussehens der Haare und der Haut.

Die moderne Kosmetik ist bemüht, das durch übermäßig starke Absonderung der Talgdrüsen und der Kopfhaut verursachte fettige und wenig ästhetische Aussehen der Haare zu vermindern. Es ist daher vielfach versucht worden, durch geeignete Mittel die Sekretion der Talgdrüsen zu normalisieren, um dem Haar wieder sein normales Aussehen zu geben. So wurden zur Bekämpfung der Seborrhoe des behaarten Kopfes kosmetische Pflegemittel mit Schwefel-, Quecksilber- oder Teerzusatz verwendet. Dabei hat sich gezeigt, daß diese Zusätze bei längerer Anwendung häufig zu Nebenwirkungen führen, ohne daß wirklich befriedigende Ergebnisse im Hinblick auf Wirksamkeit und anwendungstechnische Eigenschaften erzielt werden konnten. Mehrfach sind auch bereits Derivate von Benzoesäureestern als antiseborrhoische Zusätze für kosmetische Mittel vorgeschlagen worden (DE-OS 31 21 064, DE-OS 33 01 313), doch besteht weiterhin der Wunsch nach Mitteln mit erhöhter sebosuppresiver Wirksamkeit.

Aufgabe der Erfindung ist es, ein kosmetisches Mittel bereitzustellen, welches gegenüber den bekannten Präparaten eine verstärkte Wirkung, ohne nachteilige Folgen auf den menschlichen Körper hat.

Es wurde nun gefunden, daß bestimmte Alkoxy- oder Alkylbenzyloxy-benzoesäuren oder deren Salze hervorragende antiseborrhoische Effekte auch bei sehr geringer Dosierung aufweisen. Gegenüber den bekannten Derivaten von Benzoesäureestern ist die Wirkung überraschenderweise erheblich verbessert, was einen geringeren Mengeneinsatz in den betreffenden sebosuppressiven kosmetischen Mitteln ermöglicht.

Gegenstand der Erfindung ist die Verwendung topischer kosmetischer Mittel, die gekennzeichnet sind durch einen Gehalt an p-Alkoxy- oder p-Alkylbenzyloxy-benzoesäuren oder deren Salze der allgemeinen Formel

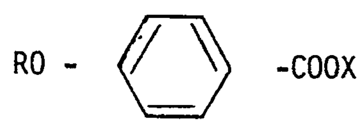

worin bedeuten

R = $C_6$-$C_{18}$-Alkyl, gerad- oder verzweigtkettig, oder
$C_3$-$C_9$-Alkylbenzyl
X = H, Alkali, Ammonium, Erdalkali, zur Behandlung von Seborrhoe.

Die anspruchsgemäß zu verwendenden Verbindungen sind zum Teil literaturbekannt und zum Teil neu.

Aus der US-A-3,716,644 ist ebenfalls bekannt, daß sich bestimmte Alkoxybenzoesäuren, deren Ester und Salze zur Senkung des Lipidgehaltes im Blutserum eignen. Ein Hinweis auf die Eignung dieser Verbindungen als Wirkstoffe zur topischen Behandlung von Seborrhoe ist der Druckschrift aber nicht zu entnehmen.

Die erfindungsgemäß zu verwendenden Verbindungen können nach allgemein bekannten Verfahren hergestellt werden, z. B. durch Alkylierung von p-Hydroxybenzoesäureestern mit Alkylhalogeniden, -sulfaten oder -sulfonaten mit Alkyl- oder Alkylbenzylresten entsprechend R und nachfolgende alkalische Hydrolyse der Alkoxy- bzw. Alkylbenzyloxybenzoesäureester. Vorzugsweise werden die Methyl- oder Ethylester eingesetzt.

Beispiele für die beanspruchten Verbindungen sind:
4-Hexyloxy-, -Heptyloxy-, -Octyloxy-, -(2,4,4-Trimethyl-pentyloxy)-(2-Ethyl-hexyloxy), -Nonyloxy-, Decyloxy-, -Undecyloxy-, -Dodecyloxy-, -Isotridecyloxy-, -Tetradecyloxy-, -Hexadecyloxyl-, -Octadecyloxy-, -Isononyloxy-, -(4-tert.-Butylbenzyloxy)-benzoesäure, sowie deren Na-, K-, Li-, $NH_4$- und Ca-Salze.

Vorzugsweise werden solche p-Alkoxy- oder -Alkylbenzyloxy-benzoesäuren oder deren Salze verwendet, bei denen der Rest R = $C_9$-$C_{14}$-Alkyl oder tert.-Butylbenzyl ist.

Die Verbindungen besitzen eine ausgeprägte sebosuppressive Wirkung bei ausgezeichneter Haut- und Schleimhautverträglichkeit. Sie lassen sich ohne Schwierigkeit in verschiedene kosmetische Zubereitungen, wie wäßrige oder alkoholische Lösungen, Öle, Suspensionen, Gele, Emulsionen, Salben oder Aerosole einarbeiten, wobei ihre größere Wasserlöslichkeit und Hydrolysestabilität gegenüber den entsprechenden Estern von besonderem Vorteil ist. Zur Behandlung von seborrhoischer Haut und fetten Haaren können diese in allen üblichen Applikationsformen, wie Haarwässern, Haarshampoos, Haarkuren, Haarspülungen,

Hautlotionen oder Schüttelmixturen eingesetzt werden. Bevorzugt ist die Verwendung in Haarpflegemitteln. Neben der erfindungsgemäßen Wirkstoffkombination können diese kosmetischen Mittel übliche Träger- und Hilfsstoffe, wie Wasser, organische Lösungsmittel, oberflächenaktive Verbindungen, Öle, Fette, Wachse, Duftstoffe, Farbstoffe, Konservierungsmittel und dergleichen enthalten. Die p-Alkoxy- oder Alkylbenzyloxy-benzoesäuren oder deren Salze werden zweckmäßig in Mengen von 0,005 - 5 Gew.-%, vorzugsweise 0,01 - 2 Gew.-%, bezogen auf das gesamte sebosuppressive Mittel, verwendet.

Herstellungsbeispiele

A) 4-Tetradecyloxy-benzoesäure

20 g Tetradecyloxy-benzoesäure-methylester wurden mit 2,75 g Natriumhydroxid in 80 ml Ethanol gelöst und nach Zusatz von 50 ml Wasser. 2,5 Stunden zum Sieden erhitzt. Nach dem Eindampfen der Suspension wurde in heißem Wasser gelöst und die Lösung mit verdünnter Salzsäure angesäuert. Nach Abfiltrieren, Waschen mit Wasser und Trocknen wurden 18,2 g (95 % d. Th.) 4-Tetradecyloxy-benzoesäure von Schmelzpunkt 95 - 99°C ( klar bei 135°C) erhalten. Analog wurden die folgenden Säuren erhalten:

B) 4-Dodecyloxy-benzoesäure

Schmelzpunkt 94 - 95°C (klar bei 135°C)

C) 4-Decyloxy-benzoesäure

Schmelzpunkt 97 - 98°C (klar bei 120°C)

D) 4-Isononyloxy-benzoesäure (neue Verbindung)

Schmelzpunkt 118 - 119°C

E) 4-(4-tert.Butyl-benzyloxy)-benzoesäure (neue Verbindung)

Schmelzpunkt 236 - 239°C

F) 4-Isotridecyloxy-benzoesäure (neue Verbindung)

Schmelzpunkt 56 °C (ab 36 °C Sintern)

Die antiseborrhoische Wirkung wurde durch nachfolgend beschriebene Tierversuche näher untersucht.

Als Versuchstiere dienten männliche Wistar-Batten mit einem Körpergewicht von 220 - 230 g zu Versuchsbeginn. Beurteilt wurde visuell der Bräunungsgrad auf dem Rücken der dort geschorenen Ratten. Die Bräunung wird durch das braune Hautoberflächenlipid der Ratten hervorgerufen. Dieser Test geht von der Beobachtung aus, daß junge weibliche Ratten sowie männliche Ratten nach dem Waschen mit Tensidlösung bzw. einem Lipidlösungsmittel und auch männliche Ratten, die systematisch mit Östrogen behandelt wurden, nur die normale helle, rosafarbene Haut nach dem Scheren aufweisen; parallel dazu sind aus den abgeschnittenen Haaren. nur noch vergleichsweise sehr geringe Lipidmengen zu extrahieren.

Zur Beurteilung der Wirksamkeit wurden die Prüfsubstanzen in alkoholischer Lösung jeweils 6 Ratten halbseitig auf das Rückenfell gepinselt. Die andere Seite wurde nur mit dem Lösungsmittel ohne Wirkstoffe behandelt.

Während der Versuchsdauer von 14 Tagen wurde an insgesamt 9 Tagen einmal appliziert. Zur weiteren Kontrolle diente eine Gruppe von 6 Ratten, die völlig unbehandelt blieben. Am Ende des Versuchs wurden die Tiere am Rücken und an den Flanken geschoren und von einem Beurteilerpanel (6 Personen) unabhängig unter Doppelblindbedingungen visuell abgemustert.

Bewertungsmethoden

Als 1. Kriterium wurde der Unterschied zwischen rechter und linker Seite gewertet, wobei pro Beurteiler und Tier jeweils 1 Punkt zu vergeben war, und zwar in der Weise, daß die

| | | |
|---|---|---|
| dunklere Seite | mit | 1 Punkt |
| hellere Seite | mit | 0 Punkte und |
| bei Gleichheit beide Seiten | mit | 0,5 Punkte |

benotet wurden.

Signifikante Differenzen zwischen unbehandelter und behandelter Seite nach dieser Bewertungsmethode zeigen die lokale Wirksamkeit einer Substanz an.

Als 2. Kriterium wurden außerdem noch die Intensitätsunterschiede der Brauntöne nach folgender Skala bewertet:

| | |
|---|---|
| 3 Punkte | stark braun |
| 2 Punkte | mittel braun |
| 1 Punkt | schwach braun |
| 0 Punkte | keine Braunfärbung. |

Nach dieser Bewertungsmethode werden die Punktsummendifferenzen zwischen den unbehandelten Kontrolltieren und jeweils den behandelten und unbehandelten Seiten ($\Delta P$)der Versuchstiere gebildet, wobei wiederum signifikante Differenzen zwischen Kontrolltieren und der behandelten Seite der Versuchstiere die Wirkung einer Substanz deutlich machen.

Parallel dazu ist in der Regel auch ein deutlicher. Unterschied zwischen der unbehandelten und der behandelten Seite der Versuchstiergruppen zu sehen. Dieser ist aber nicht immer so deutlich wie der zwischen Kontrolltieren und behandelter Seite, wofür es verschiedene Gründe geben kann, wie zum Beispiel mechanische Substanzübertragung von einer auf die andere Seite oder Lösungsmitteleinfluß.

Zur Differenzierung der Effekte gemäß Beurteilungsmethode 1 und 2 wurde das folgende Schema verwendet:

| Zeichen | Punktdifferenz |
|---|---|
| ++ | sehr groß ($\geq$ 99,9 % Wahrscheinlichkeit) |
| + | signifikant ($\geq$ 95 % Wahrscheinlichkeit) |
| - | ($<$ 95 % Wahrscheinlichkeit) |

Prozentuale Sebumreduktion

Die Sebumreduktion errechnet sich aus der Punktedifferenz in der Weise, daß man den Quotienten aus der Punktedifferenz $\Delta$ P und der Punktezahl für die Kontrolgruppe $P_k$ bildet und den erhaltenen Wert in % angibt.

$$\text{Sebumreduktion} = \frac{\Delta P}{P_k} \cdot 100 \; [\%]$$

Die p-Alkoxy- bzw. -Alkylbenzyloxy-benzoesäuren wurden in Konzentrationen von 0,01 bis 0,5 % in Alkohol in der angegebenen Weise appliziert. Die Ergebnisse sind in der Tabelle zusammengefaßt.

Tabelle

Bewertung der sebosuppressiven Effekte

| Substanz | % Sebumreduktion bei Anwendungskonzentration | | | |
|---|---|---|---|---|
| | o,1 % | 0,05 % | 0,01 % | 0,001 % |
| B | 92 | 76 | 35 | - |
| C | 42 | - | - | - |
| D | 9o | 68 | - | - |
| E | 95 | - | - | - |
| F | - | 83 | 70 | 21 |
| 4-Dodecyloxy-benzoesäuremethylester DE-OS 31 21 o64 | 18 | 0 | - | - |
| 4-Dodecyloxy-benzoesäure-2-methoxyethylester DE-OS 33 o1 313 | 22 | - | - | - |

Aus den Werten der Tabelle wird die überlegene Wirkung der beanspruchten Substanzen deutlich.

Beispiele für Rezepturen

Nachfolgend werden für die erfindungsgemäßen topischen Mittel zur Behandlung von stark fettendem Haar und seborrhoischer Haut Rezepturen gegeben:

| 1. Shampoo für fettendes Haar | Gewichtsteile |
| --- | --- |
| Ammoniumlaurylsulfat mit 33-35 % Waschaktivsubstanz | 4o,o |
| Kokosfettsäurediethanolamid | 3,o |
| Natriumchlorid | 2,o |
| Natriumsulfat | 2,o |
| 4-Dodecyloxy-benzoesäure (Substanz B) | 1,o |
| Konservierungsmittel | o,1 |
| Parfümöl | o,1 |
| Wasser | 51,8 |
| pH auf 7,2 (mit Natronlauge) | |

| 2. Haarkur | |
| --- | --- |
| Glycerinmono-distearat | o,7 |
| kationisches Tensid (z.B.Lauryltrimethylammoniumchlorid) | 2,o |
| Cholesterin | o,2 |
| Sojalecithin | o,3 |
| Emulgade A(R) (Gemisch von Cetylstearylalkohol mit nichtionogenen Emulgatoren) | 7,o |
| Parfümöl | o,3 |
| 4-Isononyloxy-benzoesäure (Substanz D) | 2,o |
| Wasser, vollentsalzt | 87,5 |
| pH 6,5 (Natronlauge) | |

## 3. Hautcreme

| | |
|---|---|
| Selbstemulgierendes Gemisch aus Mono/Diglyceriden höherer gesättigter Fettsäuren mit Kaliumstearat (Cutina KD 16(R)) | 16,o |
| Cetylstearylalkohol mit ca. 12 Mol Ethylenoxid (Eumulgin in B1(R)) | 1,o |
| 2-Octyldodecanol | 6,o |
| Isopropylmyristat | 4,o |
| Glycerin | 6,o |
| 4-tert.-Butyl-benzyl-benzoesäure (Substanz E) | o,4 |
| Konservierungsmittel (z.B. 4-Hydroxybenzoesäure-methylester) | o,1 |
| Wasser | 66,5 |

**Patentansprüche**

**1.** Verwendung von topischen kosmetischen Mitteln, gekennzeichnet durch einen Gehalt an p-Alkoxy- oder p-Alkylbenzyloxy-benzoesäuren oder deren Salzen der allgemeinen Formel

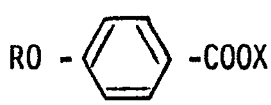

worin bedeuten,
R = $C_6$-$C_{18}$-Alkyl, gerad- oder verzweigtkettig, oder $C_3$-$C_9$-Alkylbenzyl
X = H, Alkali, Ammonium, Erdalkali,
zur Behandlung von Seborrhoe.

**2.** Verwendung nach Anspruch 1, dadurch gekennzeichnet, daß in den Verbindungen der angegebenen Formel
R = $C_9$-$C_{14}$-Alkyl, oder tert.-Butylbenzyl
ist.

**3.** Verwendung nach Anspruch 1 und 2, dadurch gekennzeichnet, daß in den Verbindungen der angegebenen Formel X = H, Li, Na, K, $NH_4$ oder Ca ist.

**4.** Verwendung nach Anspruch 1 bis 3, dadurch gekennzeichnet, daß die Verbindungen der angegebenen Formel in einer Menge von 0,005 - 5 Gew.-%, vorzugsweise 0,01 - 2 Gew.-%, bezogen auf das gesamte Mittel, enthalten sind.

5. 4-Isononyloxy-benzoesäure.

6. 4-(4-tert.-Butyl-benzyloxy)-benzoesäure.

## Claims

1. The use of topical cosmetic preparations which are characterized by a content of p-alkoxy or p-alkylbenzyloxy benzoic acids, or salts thereof, corresponding to the following general formula

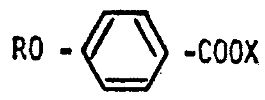

in which
R = $C_6$-$C_{18}$ alkyl, linear or branched, or $C_3$-$C_9$ alkylbenzyl,
X = H, alkali, ammonium, alkaline earth,
for the treatment of seborrhoea.

2. The use claimed in claim 1, characterized in that, in the compounds corresponding to the above formula, R = $C_9$-$C_{14}$ alkyl or tert. butyl benzyl.

3. The use claimed in claims 1 and 2, characterized in that, in the compounds corresponding to the above formula, X = H, Li, Ha, K, $NH_4$ or Ca.

4. The use claimed in claims 1 to 3, characterized in that the compounds corresponding to the above formula are present in a quantity of 0.005 to 5% by weight and preferably in a quantity of 0.01 to 2% by weight, based on the preparation as a whole.

5. 4-Isononyloxy benzoic acid.

6. 4-(4-Tert. butylbenzyloxy)-benzoic acid.

## Revendications

1. Utilisation de compositions topiques cosmétiques, caractérisée par la présence d'acides benzoïques substitués en p-alkoxy ou en p-alkylbenzyloxy ou de leurs sels de formule générale :

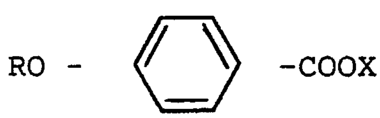

où les lettres ont les significations suivantes :
R = C6 - C18-alkyle, linéaire ou ramifié,
ou C3 - C9-alkylbenzyle,
X = H, élément alcalin, ammonium, élément alcalinoterreux, pour le traitement de la séborrhée.

2. Utilisation selon la revendication 1, caractérisée en ce que dans les composés de formule indiquée, on a :
R = C9 - C14-alkyle, ou un butylbenzyle tertiaire.

3. Utilisation selon l'une quelconque des revendications 1 et 2, caractérisée en ce que dans les composés de formule indiquée, on a :
X = H, Li, Na, K, NH4 ou Ca.

4. Utilisation selon l'une quelconque des revendications 1 à 3, caractérisée en ce que les composés de la formule indiquée sont présents à hauteur de 0,005 à 5 % en poids, de préférence entre 0,01 et 2 % en poids, exprimé par rapport au poids total de la composition cosmétique.

5. Acide benzoïque substituée en 4-isononyloxy.

6. Acide benzoïque substitué en 4-(4-butylbenzyloxy tertiaire).